# EUROPÄISCHE PATENTSCHRIFT

(11) **EP 4 591 684 B1**
(45) Veröffentlichungstag und Bekanntmachung des Hinweises auf die Patenterteilung: **05.08.2026**
(21) Anmeldenummer: 23773294.6
(22) Anmeldetag: 20.09.2023
(51) Int. Cl.: H05H 1/24

(54) **PLASMA-BEHANDLUNGSANORDNUNG**
PLASMA TREATMENT ARRANGEMENT
AGENCEMENT DE TRAITEMENT AU PLASMA

(30) Priorität: 20.09.2022 DE 102022124101
(43) Veröffentlichungstag der Anmeldung: 30.07.2025
(73) Patentinhaber: Cinogy GmbH, 37115 Duderstadt (DE)
(72) Erfinder: WANDKE, Dirk, 37308 Heilbad Heiligenstadt (DE); LETTKE, Ronny, 37115 Duderstadt (DE); SCHULZE, Sarah, 37191 Lindau (DE)
(74) Vertreter: Gramm, Lins & Partner Patent- und Rechtsanwälte PartGmbB
(86) Internationale Anmeldenummer: PCT/EP2023/075971
(87) Internationale Veröffentlichungsnummer: WO 2024/061985

(56) Entgegenhaltungen:
- CN-A- 107 320 847
- CN-A- 109 936 903
- CN-A- 115 054 832
- US-A1- 2019 327 823

## Beschreibung

Die Erfindung betrifft eine Plasma-Behandlungsanordnung zur Ausbildung einer dielektrisch behinderten Plasmaentladung mit einer Elektrodenanordnung und einem Hochspannungsgenerator.

Es ist bekannt, Oberflächen, wie beispielsweise eine Hautoberfläche von Menschen oder Tieren, mit einem Plasma zu behandeln. Dabei wird unter Atmosphärendruck eine dielektrisch behinderte Plasmaentladung erzeugt, indem eine in einem Dielektrikum eingebettete und somit zur zu behandelnden Oberfläche hin abgeschirmte Elektrode mit einer Wechselhochspannung beaufschlagt wird. Eine solche Behandlungsvorrichtung ist beispielsweise aus der DE 103 24 926 B3 bekannt. Dabei kann vorgesehen sein, dass der Körper mit der zu behandelnden Oberfläche die Masseelektrode bildet. Die Plasmabehandlung fördert die Wundheilung und dient insbesondere auch der Desinfektion der Haut von menschlichen oder tierischen Körpern.

Eine ähnliche Behandlungsvorrichtung ist auch aus der DE 10 2016 118 569 A1 bekannt, bei der die Elektrodenanordnung zur Ausbildung einer dielektrisch behinderten Plasmaentladung wenigstens zwei nebeneinander angeordnete und durch das Dielektrikum voneinander isolierte Teilelektroden hat. Die Teilelektroden werden dabei mittels einer Steuereinrichtung mit bezüglich der Wellenform und der Spannungshöhe gegengleichen, sich kompensierende Teil-Wechselhochspannungen gespeist, wobei auch hier die zu behandelnde Oberfläche als Masseelektrode dient.

Um sich verschiedenen Hautkonturen anpassen zu können, ist es des Weiteren bekannt, dass das Dielektrikum und die durch das Dielektrikum eingebettete Elektrode einer solchen Plasma-Behandlungsvorrichtung flexibel ausgestaltet sind. Aus der DE 102016 108 450 A1 ist beispielsweise eine Elektrodenanordnung bekannt, bei der das Dielektrikum und die Elektrode aus einem flexiblen Kunststoffmaterial gebildet sind, wobei die Elektrode mit elektrisch leitfähigen Zusätzen versehen ist.

Wird die zu behandelnde Hautoberfläche als Masseelektrode für die dielektrisch behinderte Plasmaentladung verwendet, so ist es ein wichtiger Aspekt sicherzustellen, dass kein ungehinderter Stromfluss im Körper des Patienten stattfindet, wenn die Elektrode der Elektrodenanordnung zur Erzeugung des dielektrisch behinderten Plasmas mit einer Wechselhochspannung gespeist wird. Ein solcher Stromfluss kann dabei beispielsweise dann stattfinden, wenn das Dielektrikum, welches zur Plasmabehandlung auf der Hautoberfläche aufliegt und diese kontaktiert, im Bereich der Elektrode derart beschädigt ist, dass bei Einspeisung der Wechselhochspannung ein Funkenschlag bzw. eine Koronaentladung in Richtung der Hautoberfläche des Patienten stattfindet. Die für die dielektrisch behinderte Plasmaentladung benötigte Wechselhochspannung liegt bspw. in einem Bereich von 12 kV bis 25 kV, wobei ein sicherer Berührungsschutz gewährleistet sein muss.

Aus der DE 20 2008 008 733 U1 ist ein Behälter, in dem zwei Elektroden zum Erzeugen von Ozon zum Sterilisieren von in dem Behälter befindlichen Lebensmitteln bekannt. Der Behälter besitzt einen Sensor, mittels dem festgestellt werden kann, ob ein Deckel des Behälters sicher geschlossen ist. Nur wenn das der Fall ist, können die Elektroden mit Spannung beaufschlagt werden.

Die DE 10 2018105 895 A1 beschreibt einen Plasmaerzeuger zum Erzeugen von Atmosphärendruck-Plasma, bei dem mittels einer Feldsonde das erzeugte magnetische Feld ermittelt wird. Anhand des gemessenen magnetischen Felds kann der piezoelektrische Transformator von einer Ansteuereinheit mit seiner Eigenfrequenz angesteuert werden, was die Effizienz der Plasmaerzeugung Effizienz steigert.

In der DE 10 2017105 430 A1 ist eine Vorrichtung zum Erzeugen eines nicht thermischen Atmosphärendruck-Plasmas bekannt, die zwei Gehäuse, nämlich für den Transformator und für die Ansteuerschaltung, verwendet. Auf diese Weise wird der Verschleiß der Ansteuerschaltung aufgrund der erzeugten reaktiven Gase vermieden.

Die DE 10 2009 011 960 A1 beschreibt ein Verfahren zum Überwachen von dielektrisch behinderten Plasmaentladungen, bei dem die durch die an die Elektroden angelegte Wechselspannung im Medium erzeugte elektrische Energie gemessen und die Signalanteile, die oberhalb einer vorgebbaren Frequenz liegen, ermittelt werden. Diese Signalanteile werden mit einer Referenz verglichen und so der Plasmaerzeugungsprozess geregelt.

Aus der DE 20 2020 104 271 U1 ist eine Vorrichtung zum Erzeugen von einer Gasentladung im Inneren eines Behandlungsinstruments bekannt, die eine Sicherheitseinrichtung umfasst, die eingerichtet ist, eine Spannung und/oder eine Frequenz hochfrequenter elektrischer Pulse an der Ausgangsseite des Transformators zum Messen. Sind die gemessenen Werte jenseits vorgegebener Grenzwert, wird die Energiezufuhr zum Transformator unterbrochen.

Die EP 3 796 362 A1 beschreibt ein Verfahren zum Erzeugen eines Plasmas in einer Plasmakammer, bei dem ein Plasmaparameter gemessen und zur Regelung der Anregungs-Spannung verwendet wird.

Die US20190327823A1 beschreibt ein Gerät zur Plasmabehandlung mit dielektrisch behindertem Plasma, bei dem Verbindungsstück, das Hochspannung führt, von dem isolierenden Dielektrikum umgeben ist, das auch die Elektrode umgibt und damit sicher gegen Berührung isoliert ist.

Die CN115054832A beschreibt ein Kosmetikgerät zur Plasmabehandlung mit je einem Schaltkreis zum Schutz vor Überhitzung und Überspannung.

Die CN 107320847A beschreibt ein Handgerät zur Tieftemperatur-Plasma-Sterilisierung mit einer Ampel-Anzeige zur Warnung bei Überschreiten der Grenzwerte für Temperatur oder elektrischen Strom.

Die CN109936903A beschreibt eine Verbesserung der Sicherheitseinrichtungen bei der Plasmaerzeugung durch Verwendung einer Abschirmung der Hochspannung mittels Teflon-Isolierung und durch einen verbesserten Überspannungsschutzschaltkreis.

Es ist daher Aufgabe der vorliegenden Erfindung, eine verbesserte Plasma-Behandlungsanordnung zur Ausbildung einer dielektrisch behinderten Plasmaentladung anzugeben, mit der insbesondere ein Fehler oder eine Fehlfunktion der Elektrodenanordnung feststellbar ist.

Die Aufgabe wird mit der Plasma-Behandlungsanordnung gemäß Anspruch 1 erfindungsgemäß gelöst. Vorteilhafte Ausgestaltungen der Erfindung finden sich in den entsprechenden Unteransprüchen.

Gemäß Anspruch 1 wird eine Plasma-Behandlungsanordnung zur Ausbildung einer dielektrisch behinderten Plasmaentladung an einer zu behandelnden Oberfläche mit einer Elektrodenanordnung und einem Hochspannungsgenerator vorgeschlagen, wobei die Elektrodenanordnung wenigstens eine Elektrode und ein die Elektrode einbettendes Dielektrikum aufweist, das die Elektrode zur zu behandelnden Oberfläche hin vollständig abdeckt. Eine solche gattungsgemäße Plasma-Behandlungsanordnung kann dabei insbesondere so ausgebildet sein, dass die zu behandelnde Oberfläche als Masseelektrode dient.

Günstig ist es, wenn die Elektrodenanordnung ausgebildet ist zum Anlegen an die zu behandelnde Oberfläche. Bei der zu behandelnden Oberfläche handelt es sich vorzugsweise um einen menschlichen oder tierischen Körper. Vorzugsweise ist die Elektrodenanordnung ausgebildet zum Anpassen an eine Oberflächenkontur des menschlichen oder tierischen Körpers. Insbesondere ist die Elektrodenanordnung ausgebildet zum Anschmiegen an den menschlichen oder tierischen Körper.

Vorzugsweise umfasst die Plasma-Behandlungsanordnung eine Befestigungsvorrichtung zum Befestigen der Elektrodenanordnung am menschlichen oder tierischen Körper.

Vorzugsweise ist die Elektrodenanordnung flexibel. Beispielsweise ist das Dielektrikum Silikon. Die wenigstens eine Elektrode kann als Metallisierung ausgebildet oder durch leitfähig gemachtes Dielektrikum, beispielsweise leitfähiges Silikon, gebildet sein.

Das Dielektrikum bildet dabei eine Anlageseite, mit der die Elektrodenanordnung der Plasma-Behandlungsanordnung auf die zu behandelnde Oberfläche aufgelegt werden kann. Die Anlageseite des Dielektrikums kann dabei strukturiert sein, um Gasräume auszubilden, in denen sich das Plasma erzeugen lässt, wenn das Dielektrikum auf der zu behandelnden Oberfläche mit der Anlageseite flächig aufliegt. So kann bspw. vorgesehen sein, dass das Dielektrikum an der Anlageseite in der Fläche und/oder am Rand Abstandshalter aufweist, zwischen denen die Gasräume ausgebildet werden. Denkbar ist aber auch, die Elektrodenanordnung auf ein (offenporiges) Textilgebilde aufzulegen, um einen Abstand zu der zu behandelnden Oberfläche auszubilden, wobei sich dann das Plasma in dem (offenporigen) Textilgebilde ausbilden kann.

Die Elektrodenanordnung kann dabei mit dem Hochspannungsgenerator mechanisch verbindbar und elektrisch kontaktierbar bzw. mechanisch verbunden und elektrisch kontaktiert ist, so dass die Elektrodenanordnung im bedarfsweise von dem Hochspannungsgenerator getrennt werden kann (bspw. beim Austausch der Elektrodenanordnung). Hierfür sind die mechanische Verbindung und die elektrische Kontaktierung lösbar ausgebildet, bspw. mithilfe einer bekannten Steckverbindung. Alternativ ist es bspw. aber auch denkbar, dass die Elektrodenanordnung mit dem Hochspannungsgenerator fest verbunden ist, so dass ein zerstörungsfreies Trennen der Elektrodenanordnung von dem Hochspannungsgenerator nicht möglich ist. Dies ist bspw. dann sinnvoll, wenn der Hochspannungsgenerator auf oder in dem Dielektrikum angeordnet ist. Dem Hochspannungsgenerator wird dabei eine Eingangsspannung zugeführt, wobei der Hochspannungsgenerator mittels mindestens eines Transformators aus der Eingangsspannung eine Wechselhochspannung generiert und diese der Elektrode der Elektrodenanordnung zur Ausbildung einer dielektrisch behinderten Plasmaentladung zuführt.

Es ist nun vorgesehen, dass die Plasma-Behandlungsanordnung eine Sicherheitseinrichtung hat, die erfindungsgemäß eine Sensoranordnung mit mindestens einem Magnetfeldsensor zum Erfassen des von dem Transformator erzeugten, elektromagnetischen (Streu-)Wechselfeldes beinhaltet und eine Auswerteeinheit aufweist, durch die das von dem Magnetfeldsensor detektierte, elektromagnetische (Streu-)Wechselfeld einem von wenigstens zwei Funktionszuständen zugeordnet wird. Insbesondere ist der erste Funktionszustand ein Soll-Zustand und der zweite Funktionszustand ein Fehlerzustand. Vorzugsweise ist die Auswerteeinheit ausgebildet zum Verbinden des Zuführens der Wechselhochspannung zur Elektrodenanordnung, wenn der Fehlerzustand detektiert wird.

Vorzugsweise wird die Sensoranordnung nicht zum Regeln der Wechselhochspannung verwendet.

Mithilfe des mindestens einen Magnetfeldsensors der Sicherheitseinrichtung wird somit das durch den Transformator erzeugte, elektromagnetische Wechselfeld erfasst. Die aus der Messung des elektromagnetischen Wechselfeldes des Transformators resultierenden Messwerte werden dann der Auswerteeinheit bspw. über eine Messwertschnittstelle bereitgestellt, wobei die Auswerteeinheit eingerichtet ist, einen Funktionszustand der Plasma-Behandlungsanordnung in Abhängigkeit von den erfassten Messwerten zu ermitteln.

So kann zur Erfassung des erzeugten, elektromagnetischen Wechselfeldes die Magnetfeldstärke erfasst werden, indem bspw. die Magnetfeldsensoren die magnetische Flussdichte messen. Die Verbindung zur magnetischen Feldstärke kann dann über die Permeabilität µ hergestellt werden, die unter anderem die Stoffeigenschaften des umgebenden Mediums berücksichtigt.

Die Auswerteeinheit kann dabei hinsichtlich des Funktionszustandes zumindest zwischen einem Normalzustand (Betriebsfall) und wenigstens einem Fehlerzustand unterscheiden und in Abhängigkeit der Messwerte des detektierten elektromagnetischen Wechselfeldes einen Normalzustand oder wenigstens einen Fehlerzustand als Funktionszustand zu ermitteln.

Mit der vorliegenden Erfindung wird es möglich, basierend auf dem elektromagnetischen Wechselfeld des Transformators insbesondere einen Fehlerzustand zu detektieren, um so die Betriebssicherheit bei der Verwendung einer derartigen Plasma-Behandlungsanordnung zu erhöhen. So ist es beispielsweise denkbar, dass bei Feststellung eines Fehlerzustandes durch die Auswerteeinheit die Sicherheitseinrichtung die gesamte Plasma-Behandlungsanordnung in einen sicheren Zustand überführt.

Basierend auf empirischen Erkenntnissen zeigt sich, dass verschiedene Funktionszustände (insbesondere verschiedene Fehlerzustände und fehlerfreier Betriebsfall) einer solchen Plasma-Behandlungsanordnung in Bezug auf die Elektrodenanordnung aus einer Erfassung des elektromagnetischen Wechselfeldes des Transformators abgeleitet werden können, da verschiedene Funktionszustände in einer entsprechenden Charakteristik des erfassten elektromagnetischen Wechselfeldes resultieren.

Durch das Auswerten des detektierten elektromagnetischen Wechselfeldes und das Erkennen von Parametern und/oder einer Charakteristik des elektromagnetischen Wechselfeldes kann auf einen entsprechenden Funktionszustand geschlossen werden. Dabei sind den verschiedenen Funktionszuständen der Plasma-Behandlungsanordnung die jeweils für die Erkennung vorgegebenen Parameter und/oder Charakteristiken des elektromagnetischen Wechselfeldes zugeordnet, sodass in Abhängigkeit des detektierten elektromagnetischen Wechselfeldes bzw. dessen Messwerte hierzu und den vorgegebenen Parametern und/oder Charakteristiken des elektromagnetischen Wechselfeldes der entsprechende Funktionszustand ermittelt werden kann.

So ist es beispielsweise denkbar, dass das detektierte elektromagnetische Wechselfeld mit den vorgegebenen Parametern und/oder Charakteristiken verglichen wird und in Abhängigkeit des Vergleiches dann ein entsprechender Funktionszustand ermittelt wird.

Gemäß einer Ausführungsform ist vorgesehen, dass die Auswerteeinheit zum Ermitteln eines Fehlerzustandes als Funktionszustand derart eingerichtet ist, dass ein Kurzschluss innerhalb der aus wenigstens zwei Teil-Elektroden bestehenden Elektrode der Elektrodenanordnung, eine fehlende elektrische Kontaktierung des Hochspannungsgenerators mit der Elektrodenanordnung und/oder ein Defekt des Dielektrikums in Abhängigkeit von dem detektierten, elektromagnetischen Wechselfeld ermittelt wird.

So kann basierend auf dem detektierten elektromagnetischen Wechselfeld bzw. den gemessenen Werten des mindestens einen Magnetfeldsensors bei einer Elektrodenanordnung mit mindestens zwei Teil-Elektroden ein Kurzschluss zwischen diesen beiden Teil-Elektroden, vorzugsweise im Bereich des Anschlussstückes, mit dem die Elektrodenanordnung mit einer Wechselhochspannungsquelle verbunden wird, festgestellt werden.

Es kann des Weiteren basierend auf dem detektierten elektromagnetischen Wechselfeld bei einer derartigen Elektrodenanordnung mit mindestens zwei Teil-Elektroden ein beidseitiger Defekt des Dielektrikums festgestellt werden, wenn durch einen Defekt des Dielektrikums ein Kurzschluss zwischen den beiden Teil-Elektroden entsteht. Es ist allerdings auch denkbar, dass nur ein einseitiger Defekt des Dielektrikums, d. h. nur im Bereich einer Teil-Elektrode oder im Bereich der Elektrode ohne Teil-Elektroden, anhand des detektierten elektromagnetischen Wechselfeldes erkannt wird. Dies kann beispielsweise aus den Parametern und/oder der Charakteristik erkennbar werden, wenn aufgrund des Defektes im Dielektrikum ein Kurzschluss mit der Masseelektrode besteht.

Des Weiteren kann basierend auf dem detektierten elektromagnetischen Wechselfeld eine fehlende elektrische Kontaktierung des Hochspannungsgenerators mit der Elektrodenanordnung festgestellt werden, beispielsweise dann, wenn die Verbindungsanordnung im Bereich des Anschlussstückes nicht ordnungsgemäß die im Dielektrikum eingebettete Elektrode elektrisch kontaktiert. Ein solcher Leerlaufzustand kann dabei beispielsweise eine Koronaentladung am Anschluss bewirken, wobei die damit einhergehende Charakteristik des detektierten elektromagnetischen Wechselfeldes diesen Fehlerzustand erkennbar macht.

Demnach ist gemäß einer Ausführungsform vorgesehen, dass die Auswerteeinheit eingerichtet ist, einen Funktionszustand der Plasma-Behandlungsanordnung aus einer Mehrzahl von vordefinierten Funktionszuständen in Abhängigkeit von dem detektierten, elektromagnetischen Wechselfeld zu ermitteln.

Gemäß einer Ausführungsform ist vorgesehen, dass die Auswerteeinheit weiterhin eingerichtet ist, einen Betriebszustand als Funktionszustand der Plasma-Behandlungsanordnung in Abhängigkeit von dem detektierten elektromagnetischen Wechselfeld zu ermitteln. So kann neben einem Fehlerzustand der Plasma-Behandlungsanordnung anhand des detektierten elektromagnetischen Wechselfeldes auch ein normaler Betriebszustand detektiert werden, der einen fehlerfreien Betrieb der Plasma-Behandlungsanordnung kennzeichnet.

Gemäß einer Ausführungsform ist vorgesehen, dass die Auswerteeinheit eingerichtet ist, erfasste Messwertverläufe, kurz Verläufe, des detektierten, elektromagnetischen Wechselfeldes mittels einer Fouriertransformation in den Frequenzraum zu transformieren und einen Funktionszustand der Plasma-Behandlungsanordnung in dem transformierten Frequenzraum zu ermitteln.

Gemäß einer Ausführungsform ist vorgesehen, dass die Sicherheitseinrichtung zum Abschalten des Hochspannungsgenerators eingerichtet ist, wenn die Auswerteeinheit einen Fehlerzustand als Funktionszustand detektiert hat. Hierfür kann vorgesehen sein, dass die Sicherheitseinrichtung über eine Abschalteinrichtung verfügt, die bei einem detektierten Fehlerzustand die Stromversorgung derart getrennt wird, dass die mindestens eine Elektrode der Elektrodenanordnung nicht mehr mit einer elektrischen Wechselhochspannung gespeist wird. Hierfür ist es denkbar, dass die elektrische Verbindung getrennt wird, bspw. mechanisch, elektrisch oder elektronisch.

Wird von der Sicherheitseinrichtung ein Fehlerzustand als Funktionszustand der Plasma-Behandlungsanordnung in Bezug auf die Elektrodenanordnung detektiert, so wird der Hochspannungsgenerator abgeschaltet. Dies bedeutet insbesondere, dass der Hochspannungsgenerator von der Eingangsspannung getrennt wird. Durch das Überführen der Plasma-Behandlungsanordnung in einen sicheren Zustand wird erreicht, dass insbesondere bei einem Defekt des Dielektrikums ein dauerhafter Kurzschluss mit der Masseelektrode vermieden wird.

Gemäß einer Ausführungsform ist vorgesehen, dass die Auswerteeinheit ein maschinelles Lernsystem hat, welches eine gelernte Korrelation zwischen auf das detektierte elektromagnetische Wechselfeld bezogenen Messwerten der Magnetfeldsensoren als Eingabedaten und Funktionszuständen der Plasma-Behandlungsanordnung als Ausgabedaten beinhaltet, wobei die Auswerteeinheit eingerichtet ist, in Abhängigkeit von durch die Sensoranordnung erfassten Messwerte als Eingabe in das maschinelle Lernsystem aus diesem einen aktuellen Funktionszustand der Plasma-Behandlungsanordnung als Ausgabe zu ermitteln.

Ein solches maschinelles Lernsystem kann dabei beispielsweise ein trainiertes künstliches neuronales Netz sein.

Die auf das detektierte elektromagnetische Wechselfeld bezogenen Messwerte der Magnetfeldsensoren, die als digitale Messwerte vorliegen und aus den analogen Werten der Magnetfeldsensoren stammen, dienen als Eingabe in das maschinelle Lernsystem. Das maschinelle Lernsystem hat dabei eine Korrelation erlernt zwischen den Messwerten und dem damit zusammenhängenden Funktionszustand der Plasma-Behandlungsanordnung, sodass durch Eingabe der digitalen Messwerte ein entsprechender Funktionszustand als Ausgabe ermittelt wird.

Zum Trainieren des maschinellen Lernsystems, insbesondere des künstlichen neuronalen Netzes, wird dem maschinellen Lernsystem eine Vielzahl von Trainingsdaten bereitgestellt, die eine Zuordnung von Messwerten oder davon abgeleiteten Werten des detektierten elektromagnetischen Wechselfeldes zu dem jeweiligen Funktionszustand für den diese Messwerte erfasst wurden, beinhalten. So kann für eine einzelne Messreihe beispielsweise für den fehlerfreien Betriebszustand als Funktionszustand ein entsprechendes Frequenzspektrum der gemessenen Werte dieser Messreihe ermittelt werden, wobei für diese Messreihe die Trainingsdaten dann das entsprechende Frequenzspektrum mit dem zugeordneten fehlerfreien Funktionszustand beinhalten. Für jeweils einen Funktionszustand werden dabei mehrere Messreihen durchgeführt, um eine möglichst breite Streuung zu erhalten.

Durch das Trainieren des maschinellen Lernsystems mit den so bereitgestellten Trainingsdaten wird durch das maschinelle Lernsystem eine Korrelation zwischen erfassten Messwerten des elektromagnetischen Wechselfeldes als Eingabedaten und Funktionszuständen der Plasma-Behandlungsanordnung als Ausgabedaten gelernt, sodass auch bei Messwerten, die von den Messwerten der Trainingsdaten abweichen, ein entsprechender korrekter Funktionszustand durch das maschinelle Lernsystem ermittelt werden kann.

Gemäß einer Ausführungsform ist vorgesehen, dass der mindestens eine Magnetfeldsensor der Sensoranordnung an der Primärseite oder der Sekundärseite des Transformators angeordnet ist.

Gemäß einer Ausführungsform ist vorgesehen, dass ein HALL-Sensor, ein AMR-Sensor und/oder eine Messspule als Magnetfeldsensor verwendet wird.

Vorzugsweise besitzt die Plasma-Behandlungsanordnung eine Ansteuereinheit, die die Sicherheitseinrichtung enthalten kann, nicht aber muss. Vorzugsweise ist die Ansteuereinheit ausgebildet zum Anlegen von pulsförmiger Wechselhochspannung an die Elektrodenanordnung. Vorzugsweise beträgt eine Pulsdauer, also die Zeit, innerhalb der pulsförmigen Wechselhochspannung auf ein Zwanzigstel ihres Maximalwerts abgefallen ist, höchstens 1 Sekunde, insbesondere höchstens 100 ms, vorzugsweise höchstens 10 ms.

Vorzugsweise ist die Auswerteeinheit ausgebildet zum automatischen Ermitteln, ob ein mittels des Magnetfeldsensors erfassten Messwerts, der eine Magnetfeldstärke des elektromagnetischen Wechselfelds beschreibt, in einem Soll-Intervall liegt. Vorzugsweise wird verneinendenfalls der Hochspannungsgenerator so angesteuert, dass dieser keine Wechselhochspannung an die Elektrodenanordnung anlegt. Alternativ oder zusätzlich wird eine Warnmeldung ausgegeben, die kodiert, dass die Elektrodenanordnung fehlerhaft ist.

Das Dielektrikum schirmt das elektrische Feld ab, das an der Elektrode der Elektrodenanordnung anliegt. Je dicker das Dielektrikum ist, desto stärker die Abschirmung. Das Soll-Intervall kann einseitig offen sein. Beispielsweise kann das Soll-Intervall eine Untergrenze enthalten und nach oben offen sein. Überschreitet der Messwert der Magnetfeldstärke die Untergrenze, so besteht das Risiko, dass das Dielektrikum fehlerhaft ist.

Vorzugsweise umfasst die Auswerteeinheit einen digitalen Speicher, in dem für zumindest eine Amplitude, insbesondere für zumindest zwei Amplituden, besonders bevorzugt für mehrere Amplituden, der Wechselhochspannung ein Soll-Intervall gespeichert ist. Je nach Amplitude der an die Elektrode angelegten Wechselhochspannung wird von der Auswerteeinheit das zugeordnete Soll-Intervall verwendet.

Die Erfindung wird anhand der beigefügten Figuren beispielhaft näher erläutert. Es zeigt
- Figur 1: eine schematische Darstellung einer Elektrodenanordnung mit zwei separaten Teil-Elektroden;
- Figur 2: ein Frequenzspektrum für den fehlerfreien Betriebsfall;
- Figur 3: ein Frequenzspektrum bei einem einseitigen Defekt des Dielektrikums;
- Figur 4: ein Frequenzspektrum bei einem beidseitigen Defekt des Dielektrikums;
- Figur 5: ein Frequenzspektrum bei einem Kurzschlussfehler;
- Figur 6: ein Frequenzspektrum bei einem Leerlauffehler.

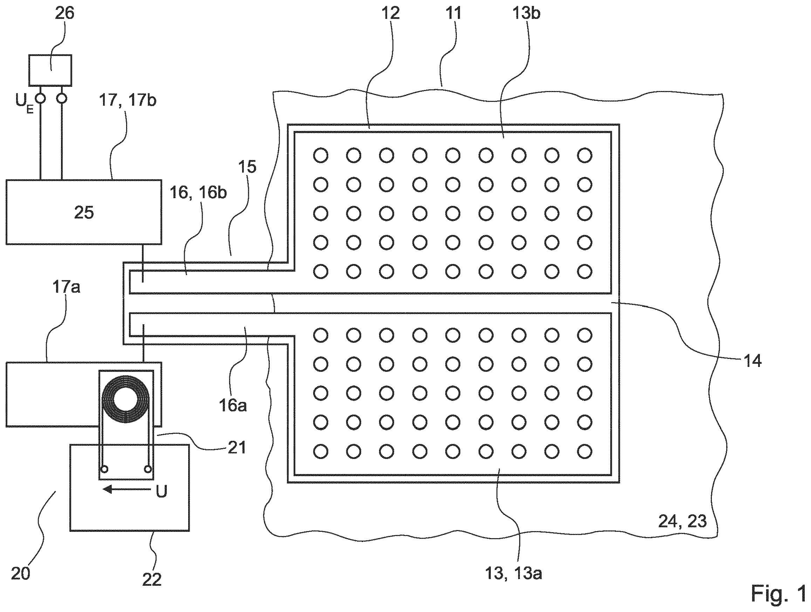

Figur 1 zeigt eine Plasma-Behandlungsanordnung 10 zur Ausbildung einer dielektrisch behinderten Plasmaentladung. Die Plasma-Behandlungsanordnung 10 weist eine Elektrodenanordnung 11 auf, die zwei separate, in einem Dielektrikum 12 eingebettete Teil-Elektroden 13 (13a, 13b) hat. In einem Mittenbereich 14 sind die beiden Teil-Elektroden 13a, 13b durch das Dielektrikum 12 voneinander isoliert. Die beiden Teil-Elektroden werden dabei mit bezüglich der Wellenform und der gegengleichen, sich kompensierenden Teil-Wechselhochspannungen gespeist.

Insbesondere ist vorgesehen, dass die beiden Teil-Elektroden für die jeweils benachbarte Teil-Elektrode keine Masseelektrode (Gegenelektrode) bildet. Vorzugsweise bildet eine zu behandelnde Oberfläche 23 eine Masseelektrode 24.

Über ein Anschlussstück 15 können die beiden Teil-Elektroden 13a, 13b mit einer Wechselhochspannungsquelle in Form eines Hochspannungsgenerators 25 verbunden werden. Der Hochspannungsgenerator 25 wird von einer Spannungsquelle 26 mit einer Eingangsspannung U_{E} versorgt. Das Anschlussstück 15 weist dabei für jede Elektrode einen elektrisch leitfähigen, beispielsweise wie hier bandförmigen, Anschlussleiter 16 auf, mit dem die jeweilige Teil-Elektrode 13a, 13b separat mit jeweils einer Wechselhochspannung U₁₃ₐ, U_{13b} beaufschlagt werden kann.

Am Ende des Anschlussstückes 15 werden die in dem Dielektrikum 12 eingebetteten Anschlussleiter 16a für die erste Teil-Elektrode 13a und 16b für die zweite Teil-Elektrode 13b durch eine Kontakteinrichtung (nicht dargestellt) kontaktiert, sodass die erste Teil-Elektrode 13a über den Anschlussleiter 16a des Anschlussstückes 15 mit einem ersten Transformator 17 in Form eines Trigger-Transformator 17a elektrisch verbunden wird, während die zweite Teil-Elektrode 13b über den Anschlussleiter 16b des Anschlussstückes 15 mit einem zweiten Transformator in Form eines Trigger-Transformators 17b elektrisch kontaktiert wird. Der Trigger-Transformator 17a und der zweite Trigger-Transformator 17b sind vorzugsweise jeweils Teil des Hochspannungsgenerators 25.

Mithilfe der Trigger-Transformatoren 17a, 17b kann die dem Wechselhochspannungsgenerator zugeführte Eingangsspannung U_{E}, die insbesondere eine Eingangswechselspannung sein kann, in die für die dielektrisch behinderte Plasmaentladung notwendigen Wechselhochspannungen U₁₃ₐ, U₁₃ₐ transformiert werden.

Die Plasma-Behandlungsanordnung 10 weist des Weiteren eine Sicherheitseinrichtung 20 auf, Sensoranordnung 27 aufweist. Die Sensoranordnung 27 besitzt einen Magnetfeldsensor 21 sowie eine Auswerteeinheit 22 hat.

Mithilfe des Magnetfeldsensors 21 wird dabei das von dem Transformator 17 beim Transformieren der Eingangswechselspannung in die gewünschte Wechselhochspannung für die Elektroden erzeugte elektromagnetische Wechselfeld erfasst. So wird beispielsweise ein Messwertverlauf Uₘ(t) erhalten. Der Messwertverlauf wird über eine Datenschnittstelle der Auswerteeinheit 22 zugeführt.

Die Auswerteeinheit 22 erhält somit von dem Magnetfeldsensor 21 eine Vielzahl von Messwerten Uₘ, die beispielsweise die Magnetfeldstärke kodieren. Bei den Messwerten Uₘ kann es sich um Spannungen handeln, das ist aber nicht notwendig. Beispielsweise kann es sich auch um elektrische Ströme handeln. Diese Messwerte Uₘ werden dabei durch die Auswerteeinheit 22 mittels einer FFT in das Frequenzspektrum umgewandelt.

Basierend auf dem Frequenzspektrum kann die Auswerteeinheit 22 nunmehr feststellen, ob eine normaler, d. h. fehlerfreie Betriebszustand bzw. Funktionszustand vorliegt oder ob ein Fehlerzustand vorliegt.

Ein Fehlerzustand kann dabei beispielsweise ein Kurzschluss in dem Anschlussstück 15 zwischen den beiden Anschlussleitern 16a, 16b sein, der sich durch eine bestimmte, von dem Normalzustand verschiedene Frequenzcharakteristik des Frequenzspektrums unterscheidet.

Beispielsweise berechnet die Auswerteeinheit 22 das Quadrat der Abweichung des normierten gemessenen Frequenzspektrums I(f) von einem vorgegebenen Soll-Frequenzspektrum Iₛₒₗₗ(f). Überschreitet diese quadratische Abweichung einen vorgegebenen Schwellenwert, steuert beispielsweise die Auswerteeinheit 22 oder eine gesonderte Steuerung so an, dass keine weitere Wechselhochspannung an die Elektrode 13 angelegt wird.

Ein Fehlerzustand kann auch der sogenannte Leerlauf sein, bei dem eine oder beide Elektroden an dem Anschlussstück 15 nicht ordnungsgemäß mit dem jeweiligen Trigger-Transformator 17 verbunden sind. Häufig kommt es hierbei zu einer Koronaentladung am Anschluss.

Ein Fehlerzustand kann dabei auch dergestalt sein, dass das Dielektrikum 12 eine Beschädigung aufweist, sodass die von dem Dielektrikum 12 abzuschirmende Elektrode 13 nicht mehr vollständig dielektrisch durch das Dielektrikum 12 abgeschirmt wird. Befindet sich ein solcher Defekt nur an einer Teil-Elektrode 13a, 13b, so entsteht oftmals einen Kurzschluss mit der Masseelektrode 24, was durch die Auswerteeinheit 22 aus dem Frequenzspektrum und der spezifischen Charakteristik für diesen Fehlerzustand ermittelbar ist. Denkbar ist aber auch, dass der Defekt des Dielektrikums 12 derart ist, dass beide Teil-Elektroden 13a, 13b davon betroffen sind, sodass entweder durch beide Teil-Elektroden 13a, 13b ein Kurzschluss mit der Masseelektrode 24 stattfindet oder ein Kurzschluss zwischen beiden Teil-Elektroden 13a, 13b entsteht. Auch in diesem Fall lässt sich anhand des spezifischen Frequenzspektrums dieses Fehlers der entsprechende Fehlerzustand ermitteln.

Basierend auf dem Frequenzspektrum der durch den Magnetfeldsensoren 21 ermittelten Messwerte wird diesem Frequenzspektrum nun ein Funktionszustand zugeordnet, welche der Charakteristik des gemessenen Frequenzspektrums am nächsten kommt.

Die Sicherheitseinrichtung 20 kann dabei so ausgebildet sein, dass sie bei einem erkannten Fehlerzustand die Stromzufuhr zu den Elektroden trennt, um so die Plasma-Behandlungsanordnung in einen sicheren Zustand zu überführen. Insbesondere dann, wenn ein Defekt im Dielektrikum vorhanden ist, muss die Sicherheitseinrichtung 20 sofort in den sicheren Zustand wechseln, um den Kurzschluss mit der Masseelektrode und somit mit der zu behandelnden Oberfläche unter allen Umständen weiter zu verhindern.

Die Auswerteeinheit 22 kann dabei eine Datenverarbeitungseinrichtung sein, die eine Messwertschnittstelle aufweist, um die von den Magnetfeldsensoren 21 aufgenommenen analogen Messwerte des elektromagnetischen Wechselfeldes empfangen zu können. Die Messwertschnittstelle steht dabei mit einem Messsignalverstärker in Verbindung, um die analogen Signale in der gewünschten Art und Weise zu verstärken. Mithilfe eines nach geschalteten AD-Wandlers werden die aufgenommenen analogen Messwerte dann in digitale Signale umgewandelt, sodass die Auswerteeinheit 22 diese dann entsprechend auswerten kann.

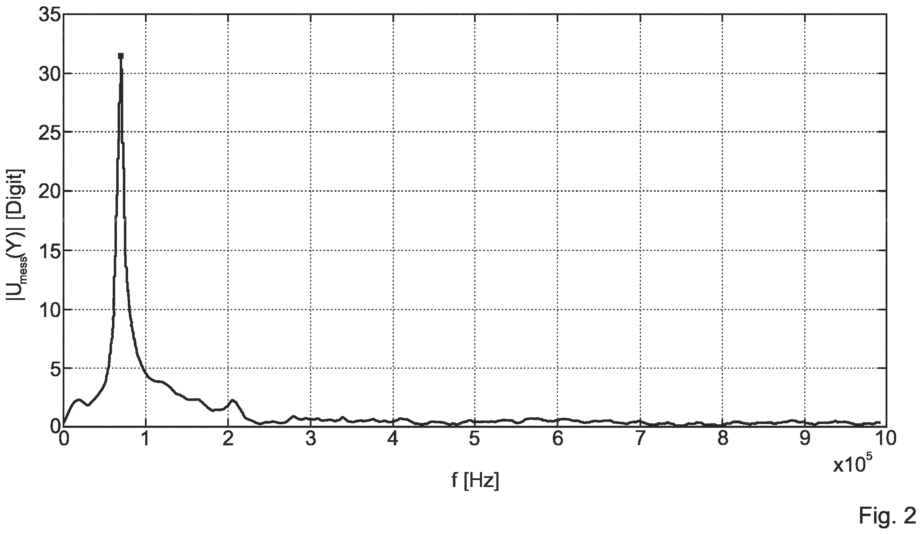

Die Figuren 2 bis 6 zeigen ein entsprechendes Frequenzspektrum für verschiedene Funktionszustände. Hier wie in allen anderen Fällen könnte statt Frequenzspektrum auch vom Amplitudenspektrum gesprochen werden da jeweils die Amplitude der entsprechenden Frequenz gegen die Frequenz aufgetragen ist. Die Daten beziehen sich dabei auf eine Elektrodenanordnung, welches leitfähiges Silikon als Elektrodenmaterial besitzt, das vollständig in einem Dielektrikum eingebettet ist.

Figur 2 zeigt das mittels FFT berechnete einseitige Frequenzspektrum in linearer Darstellung der AD-gewandelten, gemessenen, induzierten Spannung in der Messspule für den normalen Betriebsfall. Es wird somit der Soll-Betriebszustand dargestellt.

Mittels der Magnetfeldsensoren wird das elektromagnetische Wechselfeld in seinem zeitlichen Verlauf erfasst und in eine analoge elektrische Meßgröße umgewandelt. Besteht der Magnetfeldsensoren beispielsweise aus einer Induktionsspule, so kann an deren beiden Enden eine elektrische Spannung gemessen werden. Mithilfe einer geeigneten Elektronik (Analog-Digital-Wandlung) wird diese analoge Messgröße, beispielsweise die erfasste elektrische Spannung, in ein digitales Signal umgewandelt, in dem das analoge Signal in zeitdiskreten Abständen abgetastet wird. Das digitale Signal, welches den zeitlichen Verlauf der analogen elektrischen Messgröße (beispielsweise Spannung) beinhaltet, wird dann einer FFT- Auswertung zugeführt, mit dem das in Figur 2 zu sehende Frequenzspektrum beispielhaft berechnet werden kann.

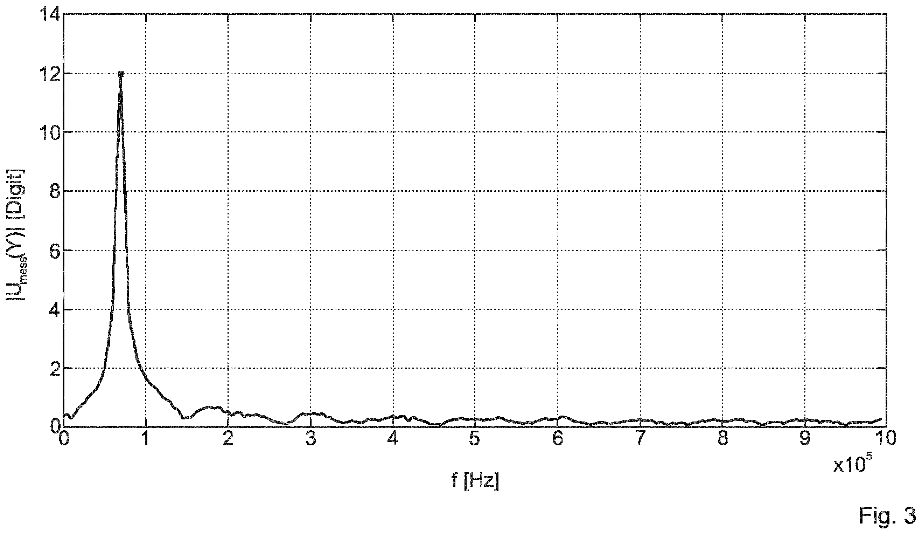

Figur 3 zeigt das mittels FFT berechnete einseitige Frequenzspektrum in linearer Darstellung der AD-gewandelten, gemessenen, induzierten Spannung in der Messspule für den einseitigen Defekt des Dielektrikums, d.h. das Dielektrikum ist im Bereich nur einer Teil-Elektrode einer zwei Teil-Elektroden aufweisenden Elektrodenanordnung defekt und bietet keine hinreichende dielektrische Abschirmung mehr.

Bei einem direkten Vergleich des Frequenzspektrums der Figur 2 bezüglich des fehlerfreien Betriebszustandes mit dem Frequenzspektrum der Figur 3 wird ersichtlich, dass die Hauptfrequenz identisch ist, während der Unterschied in der Amplitude besteht, deren Spitzenwert in Figur 3 ungefähr die Hälfte des Spitzenwertes der Figur 2 beträgt.

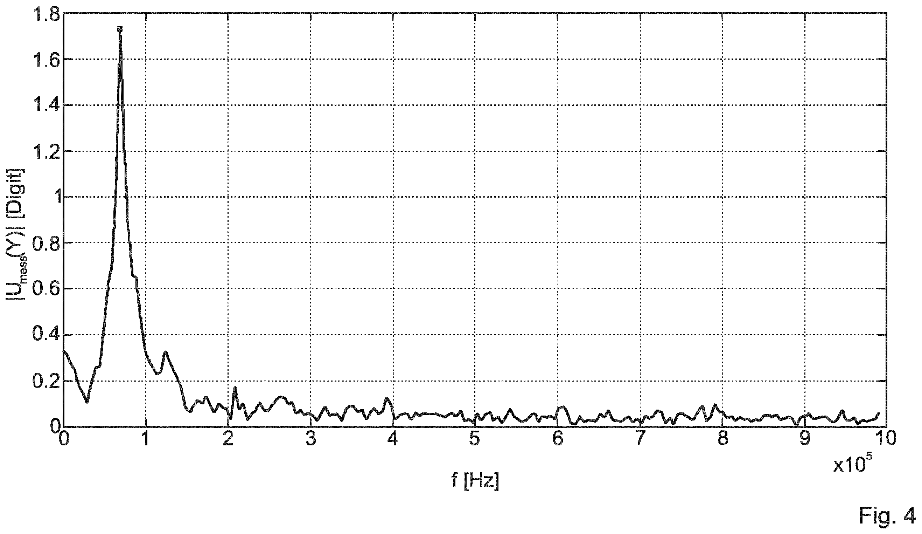

Figur 4 zeigt das mittels FFT berechnete einseitige Frequenzspektrum in linearer Darstellung der AD-gewandelten gemessenen, induzierten Spannung in der Messspule für den beidseitigen Defekt des Dielektrikums, d. h. das Dielektrikum ist im Bereich beider Teil-Elektroden einer zwei Teil-Elektroden aufweisenden Elektrodenanordnung defekt und bietet keine hinreichende dielektrische Abschirmung mehr für beide TeilElektroden.

Im Vergleich zu den Amplituden der Figuren 2 und 3 ist die Hauptfrequenz schmalbandiger ausgeprägt und besitzt lediglich eine Amplitude von weniger als zwei Digit. Das bedeutet, dass zwischen den Amplituden aus Figur 3 und 4 ein Faktor größer sechs vorliegt. Dabei kann der erkennbar schmalbandigere Peak der Hauptfrequenz gegebenenfalls ergänzend ausgewertet werden.

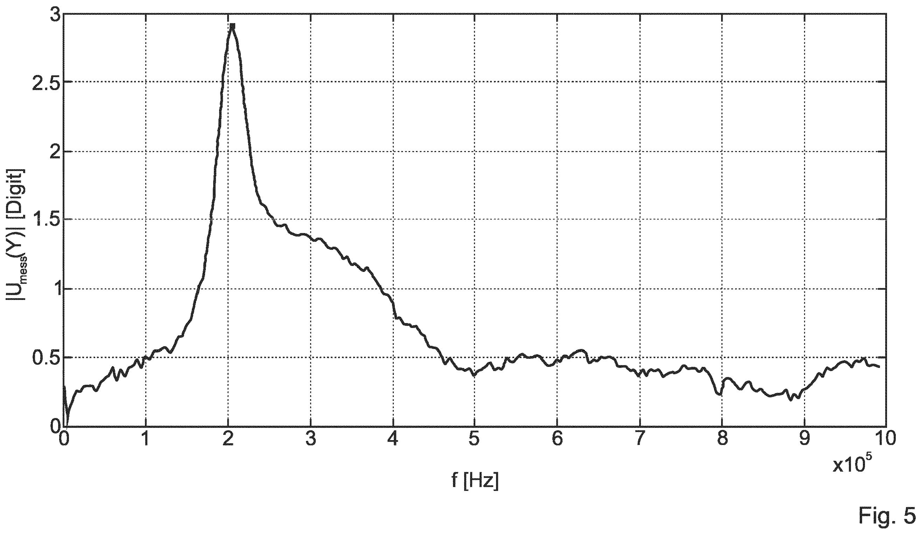

Figur 5 zeigt das mittels FFT berechnete einseitige Frequenzspektrum in linearer Darstellung der AD-gewandelten, gemessenen, induzierten Spannung in der Messspule für den Kurzschluss zwischen den Teil-Elektroden einer zwei Teil-Elektroden aufweisenden Elektrodenanordnung. Im Vergleich zu den vorherigen Amplitudenspektren ist die Hauptfrequenz verschoben und liegt bei ungefähr 200 kHz. Dies entspricht einer Frequenzänderung größer Faktor zwei bis drei gegenüber der Hauptfrequenz vom fehlerfreien Betriebszustand.

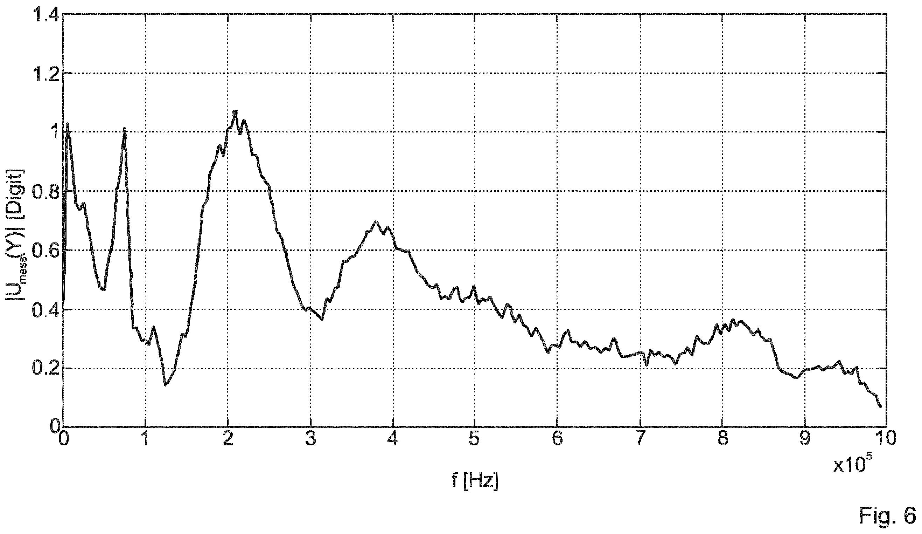

Figur 6 zeigt das mittels FFT berechnete einseitige Frequenzspektrum in linearer Darstellung der AD-gewandelten, gemessenen, induzierten Spannung in der Messspule für den Leerlauf, d. h. es ist keine Elektrodenanordnung elektrisch kontaktiert. Das bedeutet, dass im Anschlussbereich der beiden Teil-Elektroden eine Funken- oder Koronaentladung, also ein elektrischer Überschlag zwischen beiden Teil-Wechselhochspannungen, stattfindet, weil die beiden Kontaktierungen offen liegen, wenn die Elektrodenanordnung sie nicht abdeckt und so über den Luftabstand ein Überschlag stattfinden kann.

Ein Vergleich zwischen den Figuren 5 und 6 zeigt, dass die Hauptfrequenz identisch ist (ca. 200 kHz), die Amplituden sich aber um mindestens den Faktor zwei, nahezu Faktor 3, unterscheiden. Weiterhin existieren in dem Frequenzspektrum der Figur 6 Nebenmaxima. Unterhalb von 100 kHz existieren Frequenzen mit Amplituden im ähnlichen Wertebereich, wie die Amplitude bei der Hauptfrequenz. Auch die Information über mehrere, mit gleicher Intensität auftretenden Frequenzen können hier als Unterscheidungskriterium herangezogen werden.

Anhand der hier beschriebenen Fälle werden zur Unterscheidung der Fälle mindestens zwei Parameter benötigt. Auf der einen Seite ist das die Amplitude und auf der anderen Seite ist das die (Haupt-)Frequenz. Die Auswertung kann dabei bspw. zuerst nach der Grundfrequenz suchen und unterscheiden. Anschließend kann dann mit der Information über die Amplitude der jeweilige Zustand eindeutig bestimmt werden.

### Bezugszeichenliste

| | | | |
|---|---|---|---|
| 10 | Plasma-Behandlungsanordnung | 20 | Sicherheitseinrichtung |
| 11 | Elektrodenanordnung | 21 | Magnetfeldsensor |
| 12 | Dielektrikum | 22 | Auswerteeinheit |
| 13 | Elektrode | 23 | zu behandelnde Oberfläche |
| 13a | erste Teil-Elektrode | 24 | Masseelektrode |
| 13b | zweite Teil-Elektrode | 25 | Hochspannungsgenerator |
| 14 | Mittenbereich | 26 | Spannungsquelle |
| 15 | Anschlussstück | 27 | Sensoranordnung |
| 16 | Anschlussleiter | | |
| 16a | erster Anschlussleiter der ersten Teil-Elektrode | f | Frequenz |
| | | I | Intensität |
| 16b | zweiter Anschlussleiter der zweiten Teil-Elektrode | I(f) | Frequenzspektrum |
| | | t | Zeit |
| 17 | Transformator | U₁₃ₐ | Wechselhochspannung |
| 17a | erster Transformator für die erste Teil-Elektrode | U_{13b} | Wechselhochspannung |
| | | U_{E} | Eingangsspannung |
| 17b | zweiter Transformator für die zweite Teil-Elektrode | Uₘ | Messwert |
| | | Uₘ(t) | Messwertverlauf |

## Patentansprüche

1. Plasma-Behandlungsanordnung (10) zur Ausbildung einer dielektrisch behinderten Plasmaentladung mit einer Elektrodenanordnung (11) und einem Hochspannungsgenerator,
(a) wobei die Elektrodenanordnung (11) wenigstens eine Elektrode (13) und ein die Elektrode (13) einbettendes Dielektrikum aufweist, das die Elektrode (13) zu einer zu behandelnden Oberfläche (23) hin vollständig abdeckt,
(b) wobei der Hochspannungsgenerator (25) mittels mindestens eines Transformators (17a, 17b) aus einer dem Hochspannungsgenerator (25) zugeführten Eingangsspannung (U_{E}) eine Wechselhochspannung (U₁₃ₐ, U_{13b}) generiert und diese der Elektrode (13) der Elektrodenanordnung (11) zur Ausbildung einer dielektrisch behinderten Plasmaentladung zuführt,
(c) wobei die Plasma-Behandlungsanordnung (10) eine Sicherheitseinrichtung (20) hat,
**dadurch gekennzeichnet, dass**
die Sicherheitseinrichtung (20)
(i) eine Sensoranordnung (27) mit mindestens einem Magnetfeldsensor (27) zum Erfassen des von dem Transformator (17a, 17b) erzeugten, elektromagnetischen Wechselfeldes umfasst und
(ii) eine Auswerteeinheit (22) aufweist, durch die das von dem Magnetfeldsensor (27) detektierte, elektromagnetische Wechselfeld einem von wenigstens zwei Funktionszuständen zugeordnet wird.

2. Plasma-Behandlungsanordnung (10) nach Anspruch 1, wobei
(a) die Elektrodenanordnung (11) ausgebildet ist zum Anlegen an die zu behandelnde Oberfläche (23) und
(b) ein Funktionszustand ein Fehlerzustand ist und die Auswerteeinheit ausgebildet zum Verhindern des Zuführens der Wechselhochspannung zur Elektrodenanordnung, wenn der Fehlerzustand detektiert wird.

3. Plasma-Behandlungsanordnung (10) nach einem der vorstehenden Ansprüche, wobei
die Auswerteeinheit (22) zum Ermitteln eines Fehlerzustandes als Funktionszustand derart eingerichtet ist, dass
(a) ein Kurzschluss innerhalb der aus wenigstens zwei Teil-Elektroden (13a, 13b) bestehenden Elektrode (13) der Elektrodenanordnung (11),
(b) eine fehlende elektrische Kontaktierung des Hochspannungsgenerators (25) mit der Elektrodenanordnung (11) und/oder
(c) ein Defekt des Dielektrikums (12) in Abhängigkeit von dem detektierten, elektromagnetischen Wechselfeld ermittelt wird.

4. Plasma-Behandlungsanordnung (10) nach einem der vorstehenden Ansprüche, wobei
die Auswerteeinheit (22) eingerichtet ist, einen fehlerfreien Betriebszustand als Funktionszustand der Plasma-Behandlungsanordnung (10) in Abhängigkeit von dem detektierten, elektromagnetischen Wechselfeld zu ermitteln.

5. Plasma-Behandlungsanordnung (10) nach einem der vorhergehenden Ansprüche, wobei die Auswerteeinheit (22) eingerichtet ist, erfasste Messwertverläufe des detektierten, elektromagnetischen Wechselfeldes in ein Frequenzspektrum zu transformieren und einen Funktionszustand der Plasma-Behandlungsanordnung (10) in Abhängigkeit von dem transformierten Frequenzspektrum zu ermitteln.

6. Plasma-Behandlungsanordnung (10) nach einem der vorhergehenden Ansprüche, wobei die Auswerteeinheit (22) eingerichtet ist, einen Funktionszustand der Plasma-Behandlungsanordnung (10) aus einer Mehrzahl von vordefinierten Funktionszuständen in Abhängigkeit von dem detektierten, elektromagnetischen Wechselfeld zu ermitteln.

7. Plasma-Behandlungsanordnung (10) nach einem der vorhergehenden Ansprüche, wobei die Sicherheitseinrichtung (20) zum Abschalten des Hochspannungsgenerators (25) eingerichtet ist, wenn die Auswerteeinheit (22) einen Fehlerzustand als Funktionszustand detektiert hat.

8. Plasma-Behandlungsanordnung (10) nach einem der vorhergehenden Ansprüche, wobei die Auswerteeinheit (22) ein maschinelles Lernsystem hat, welches eine gelernte Korrelation zwischen auf das detektierte elektromagnetische Wechselfeld bezogenen Messwerten der Magnetfeldsensoren als Eingabedaten und Funktionszuständen der Plasma-Behandlungsanordnung (10) als Ausgabedaten beinhaltet, wobei die Auswerteeinheit (22) eingerichtet ist, in Abhängigkeit von durch die Sensoranordnung (27) erfassten Messwerte als Eingabe in das maschinelle Lernsystem aus diesem einen aktuellen Funktionszustand der Plasma-Behandlungsanordnung (10) als Ausgabe zu ermitteln.

9. Plasma-Behandlungsanordnung (10) nach Anspruch 8, wobei das maschinelle Lernsystem ein trainiertes künstliches neuronales Netz ist.

10. Plasma-Behandlungsanordnung (10) nach einem der vorhergehenden Ansprüche, wobei der mindestens eine Magnetfeldsensor (27) der Sensoranordnung (27) an der Primärseite oder der Sekundärseite des Transformators angeordnet ist.

11. Plasma-Behandlungsanordnung (10) nach einem der vorhergehenden Ansprüche, wobei ein HALL-Sensor, ein AMR-Sensor und/oder eine Messspule als Magnetfeldsensoren verwendet werden.

12. Plasma-Behandlungsanordnung (10) nach einem der vorhergehenden Ansprüche, wobei
(a) sie so ausgebildet ist, dass die zu behandelnde Oberfläche als Masseelektrode dient und
(b) die Sicherheitseinrichtung (20) so ausgebildet ist, dass
ein Funkenüberschlag und/oder
eine Koronaentladung
zwischen Elektrodenanordnung (11) und der zu behandelnden Oberfläche (23) oder zwischen den Teil-Elektroden (13a, 13b) vermieden wird.

13. Plasma-Behandlungsanordnung (10) nach einem der vorhergehenden Ansprüche, wobei
sie so ausgebildet ist, dass beim Anlegen der Elektrodenanordnung (11) an die zu behandelnde Oberfläche und Anlegen der Wechselhochspannung an die Elektrodenanordnung (11) zwischen der Elektrodenanordnung (11) und der zu behandelnden Oberfläche ein Plasma erzeugt wird.

14. Plasma-Behandlungsanordnung (10) nach einem der vorhergehenden Ansprüche, wobei
die Auswerteeinheit (22) ausgebildet ist zum automatischen Ermitteln, ob ein mittels des Magnetfeldsensors erfasster Messwert, der eine Magnetfeldstärke des elektromagnetischen Wechselfelds beschreibt, in einem Soll-Intervall liegt, und verneinendenfalls Ansteuern des Hochspannungsgenerators (25), sodass keine Wechselhochspannung an die Elektrodenanordnung (11) anliegt.

## Claims

1. A plasma treatment arrangement (10) for forming a dielectric barrier plasma discharge, comprising an electrode arrangement (11) and a high-voltage generato r,
(a) the electrode arrangement (11) comprising at least one electrode (13) and a dielectric embedding the electrode (13), said dielectric completely covering the electrode (13) towards the surface to be treated (23),
(b) wherein the high-voltage generator (25) generates an alternating high voltage (U₁₃ₐ, U_{13b}) by means of at least one transformer (17a, 17b) from an input voltage (U_{E}) fed to the high-voltage generator (25) and feeds it to the electrode (13) of the electrode arrangement (11) for forming a dielectric barrier plasma discharge,
(c) wherein the plasma treatment arrangement (10) has a safety device (20)
**characterized in that**
the safety device (20)
(i) includes a sensor arrangement (27) with at least one magnetic field sensor (27) for detecting the alternating electromagnetic field generated by the transformer (17a, 17b) and
(ii) comprises an evaluation unit (22) by way of which the alternating electromagnetic field detected by the magnetic field sensor (27) is allocated to one of at least two functional states.

2. The plasma treatment arrangement (10) according to claim 1, wherein
(a) the electrode arrangement (11) is designed to be applied to the surface to be treated (23) and
(b) a functional state is an error state and the evaluation unit is configured to prevent the supply of the alternating high voltage to the electrode arrangement when the error state is detected.

3. The plasma treatment arrangement (10) according to one of the preceding claims, wherein
the evaluation unit (22) is configured to detect an error state as a functional state in such a way that
(a) a short circuit within the electrode (13) of the electrode arrangement (11), said electrode comprising at least two partial electrodes (13a, 13b),
(b) a lack of electrical contacting of the high-voltage generator (25) to the electrode arrangement (11) and/or
(c) a defect of the dielectric (12) is determined as a function of the alternating electromagnetic field detected.

4. The plasma treatment arrangement (10) according to one of the preceding claims, wherein
the evaluation unit (22) is configured to determine an error-free operating state as a functional state of the plasma treatment arrangement (10) as a function of the alternating electromagnetic field detected.

5. The plasma treatment arrangement (10) according to one of the preceding claims, wherein the evaluation unit (22) is configured to transform detected measured value progressions of the alternating electromagnetic field detected into a frequency spectrum and to determine a functional state of the plasma treatment arrangement (10) as a function of the transformed frequency spectrum.

6. The plasma treatment arrangement (10) according to one of the preceding claims, wherein the evaluation unit (22) is configured to determine a functional state of the plasma treatment arrangement (10) from a plurality of predefined functional states as a function of the alternating electromagnetic field detected.

7. The plasma treatment arrangement (10) according to one of the preceding claims, wherein the safety device (20) is configured to switch off the high-voltage generator (25) if the evaluation unit (22) has detected an error state as a functional state.

8. The plasma treatment arrangement (10) according to one of the preceding claims, wherein the evaluation unit (22) has a machine learning system that contains a learned correlation between measured values of the magnetic field sensors related to the detected alternating electromagnetic field as input data and functional states of the plasma treatment arrangement (10) as output data, wherein the evaluation unit (22) is configured to determine a current functional state of the plasma treatment arrangement (10) as output from the machine learning system as a function of measured values detected by the sensor arrangement (27) as input to the machine learning system.

9. The plasma treatment arrangement (10) according to claim 8, wherein the machine learning system is a trained artificial neuronal network.

10. The plasma treatment arrangement (10) according to one of the preceding claims, wherein that the at least one magnetic field sensor (27) of the sensor arrangement (27) is arranged on the primary or secondary side of the transformer.

11. The plasma treatment arrangement (10) according to one of the preceding claims, wherein a HALL sensor, an AMR sensor and/or a measuring coil are used as a magnetic field sensors.

12. The plasma treatment arrangement (10) according to one of the preceding claims, wherein
(a) it is designed in such a way that the surface to be treated acts as a ground electrode and
(b) the safety device (20) is designed in such a way that
a spark-over and/or
a corona discharge
between the electrode arrangement (11) and the surface to be treated (23) or between the partial electrodes (13a, 13b) is avoided.

13. The plasma treatment arrangement (10) according to one of the preceding claims, wherein
it is designed such that a plasma is generated between the electrode arrangement (11) and the surface to be treated when the electrode arrangement (11) is applied to the surface to be treated and the alternating high voltage is applied to the electrode arrangement (11).

14. The plasma treatment arrangement (10) according to one of the preceding claims, wherein
the evaluation unit (22) is configured to automatically determine whether a measured value detected by means of the magnetic field sensor, wherein the measured value describes the magnetic field strength of the alternating electromagnetic field, lies within a target interval and, if not, to control the high-voltage generator (25) in such a way that an alternating high voltage is not applied to the electrode arrangement.

## Revendications

1. Dispositif de traitement par plasma (10) destiné à engendrer une décharge de plasma à barrière diélectrique, comprenant un ensemble d'électrode (11) et un générateur de haute tension, dans lequel
(a) l'ensemble d'électrode (11) comprend au moins une électrode (13) et un diélectrique enrobant l'électrode (13), lequel recouvre entièrement l'électrode (13) en direction d'une surface à traiter (23),
(b) au moyen d'au moins un transformateur (17a, 17b), le générateur de haute tension (25) génère, à partir d'une tension d'entrée (U_{E}) fournie au générateur de haute tension (25), une haute tension alternative (U₁₃ₐ, U_{13b}) et l'applique à l'électrode (13) de l'ensemble d'électrode (11) pour former une décharge de plasma à barrière diélectrique,
(c) le dispositif de traitement par plasma (10) comportant un dispositif de sécurité (20),
**caractérisé en ce que**
le dispositif de sécurité (20) comprend
(i) un ensemble de capteur (27) comportant au moins un capteur de champ magnétique (27) destiné à détecter le champ électromagnétique alternatif généré par le transformateur (17a, 17b), et
(ii) une unité d'évaluation (22) par laquelle le champ électromagnétique alternatif, détecté par le capteur de champ magnétique (27), est associé à l'un d'au moins deux états de fonctionnement.

2. Dispositif de traitement par plasma (10) selon la revendication 1, dans lequel
(a) l'ensemble d'électrode (11) est conçu pour être appliqué sur la surface à traiter (23), et
(b) un état de fonctionnement est un état de défaut, et l'unité d'évaluation est conçue pour empêcher l'alimentation en haute tension alternative de l'ensemble d'électrode lorsque l'état de défaut est détecté.

3. Dispositif de traitement par plasma (10) selon l'une des revendications précédentes, dans lequel, pour déterminer un état de défaut en tant qu'état de fonctionnement, l'unité d'évaluation (22) est conçue de manière à déterminer
(a) un court-circuit à l'intérieur de l'électrode (13) de l'ensemble d'électrode (11), constituée d'au moins deux électrodes partielles (13a, 13b),
(b) une absence de contact électrique entre le générateur de haute tension (25) et l'ensemble d'électrode (11), et/ou
(c) un défaut du diélectrique (12), en fonction du champ électromagnétique alternatif détecté.

4. Dispositif de traitement par plasma (10) selon l'une des revendications précédentes, dans lequel
l'unité d'évaluation (22) est conçue pour déterminer un état de fonctionnement sans défaut comme état de fonctionnement du dispositif de traitement par plasma (10), en fonction du champ électromagnétique alternatif détecté.

5. Dispositif de traitement par plasma (10) selon l'une des revendications précédentes, dans lequel l'unité d'évaluation (22) est conçue pour transformer les courbes de valeurs de mesure enregistrées du champ électromagnétique alternatif détecté en un spectre de fréquences et pour déterminer un état de fonctionnement du dispositif de traitement par plasma (10) en fonction du spectre de fréquences transformé.

6. Dispositif de traitement par plasma (10) selon l'une des revendications précédentes, dans lequel l'unité d'évaluation (22) est conçue pour déterminer un état de fonctionnement du dispositif de traitement par plasma (10) parmi une pluralité d'états de fonctionnement prédéfinis, en fonction du champ électromagnétique alternatif détecté.

7. Dispositif de traitement par plasma (10) selon l'une des revendications précédentes, dans lequel le dispositif de sécurité (20) est conçu pour couper le générateur de haute tension (25) lorsque l'unité d'évaluation (22) a détecté un état de défaut comme état de fonctionnement.

8. Dispositif de traitement par plasma (10) selon l'une des revendications précédentes, dans lequel l'unité d'évaluation (22) comporte un système d'apprentissage automatique qui contient, en tant que données d'entrée, une corrélation apprise entre des valeurs de mesure des capteurs de champ magnétique, relatives au champ électromagnétique alternatif détecté, et, en tant que données de sortie, des états de fonctionnement du dispositif de traitement par plasma (10), et l'unité d'évaluation (22) est conçue pour déterminer, en fonction de valeurs de mesure enregistrées par l'ensemble de capteur (27) en tant qu'entrée dans le système d'apprentissage automatique, un état de fonctionnement actuel du dispositif de traitement par plasma (10) en tant que sortie dudit système.

9. Dispositif de traitement par plasma (10) selon la revendication 8, dans lequel le système d'apprentissage automatique est un réseau neuronal artificiel entraîné.

10. Dispositif de traitement par plasma (10) selon l'une des revendications précédentes, dans lequel ledit au moins un capteur de champ magnétique (27) de l'ensemble de capteur (27) est disposé du côté primaire ou du côté secondaire du transformateur.

11. Dispositif de traitement par plasma (10) selon l'une des revendications précédentes, dans lequel un capteur à effet Hall, un capteur AMR (magnétorésistance anisotrope) et/ou une bobine de mesure sont utilisés comme capteurs de champ magnétique.

12. Dispositif de traitement par plasma (10) selon l'une des revendications précédentes, dans lequel
(a) il est conçu de telle sorte que la surface à traiter sert d'électrode de masse, et
(b) le dispositif de sécurité (20) est conçu de manière à éviter
un amorçage d'arc et/ou
une décharge en couronne
entre l'ensemble d'électrode (11) et la surface à traiter (23) ou entre les électrodes partielles (13a, 13b).

13. Dispositif de traitement par plasma (10) selon l'une des revendications précédentes, dans lequel
il est conçu de telle sorte que, lors de l'application de l'ensemble d'électrode (11) à la surface à traiter et lors de l'application de la haute tension alternative à l'ensemble d'électrode (11), un plasma est généré entre l'ensemble d'électrode (11) et la surface à traiter.

14. Dispositif de traitement par plasma (10) selon l'une des revendications précédentes, dans lequel
l'unité d'évaluation (22) est conçue pour déterminer automatiquement si une valeur de mesure enregistrée par le capteur de champ magnétique, laquelle décrit l'intensité de champ magnétique du champ électromagnétique alternatif, se situe dans un intervalle de consigne, et, dans le cas contraire, commander le générateur de haute tension (25) de telle sorte qu'aucune haute tension alternative ne soit appliquée à l'ensemble d'électrode (11).
